(19) Europäisches Patentamt
European Patent Office
Office européen des brevets

(11) EP 0 887 339 A1

# (12) EUROPÄISCHE PATENTANMELDUNG

(43) Veröffentlichungstag:
**30.12.1998 Patentblatt 1998/53**

(51) Int. Cl.$^6$: **C07C 229/50**, C07C 233/52, C07C 233/91, C07C 237/18

(21) Anmeldenummer: **97110533.3**

(22) Anmeldetag: **27.06.1997**

(84) Benannte Vertragsstaaten:
**AT BE CH DE DK ES FI FR GB GR IE IT LI LU MC NL PT SE**
Benannte Erstreckungsstaaten:
**AL LT LV RO SI**

(71) Anmelder:
**BOEHRINGER MANNHEIM GMBH**
**68298 Mannheim (DE)**

(72) Erfinder:
- **Dickhaut, Joachim, Dr., Dipl.-Chemiker 69121 Heidelberg (DE)**
- **Grams, Frank, Dr., Dipl.-Chemiker 68219 Mannheim (DE)**
- **Haag, Rainer, Dr., Dipl.-Chemiker 68526 Ladenburg (DE)**

Bemerkungen:
Ein Antrag gemäss Regel 88 EPÜ auf Berichtigung der Beschreibung, der Patentansprüche sowie der Zusammenfassung liegen vor. Über diesen Antrag wird im Laufe des Verfahrens vor der Prüfungsabteilung eine Entscheidung getroffen werden (Richtlinien für die Prüfung im EPA, A-V, 3.).

## (54) Neue Azulenderivate und diese enthaltende Arzneimittel

(57) Gegenstand der vorliegenden Erfindung sind neue Azulenderivate der allgemeinen Formeln I bzw. II.

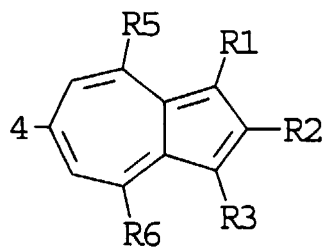

I

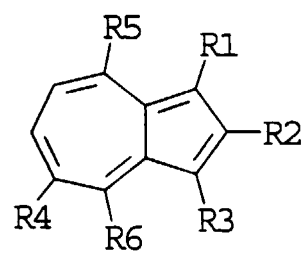

II

wobei $R_1$ bis $R_6$ die in der Beschreibung angegebene Bedeutung haben sowie deren Tautomere, Enantiomere, Diastereomere, Racemate und physiologisch verträglichen Salze oder Ester und Substanzen, die *in vivo* zu Verbindungen der Formel I bzw. II hydrolysiert oder metabolisiert werden.
Die Erfindung betrifft auch Verfahren zur Herstellung der obigen Verbindungen, Arzneimittel, die solche Verbindungen enthalten, sowie die Verwendung dieser Verbindungen bei der Herstellung von Arzneimitteln mit Metalloproteinaseinhibierender Wirkung.

EP 0 887 339 A1

## Beschreibung

Die vorliegende Erfindung betrifft neue Azulenderivate der allgemeinen Formel I bzw. II.

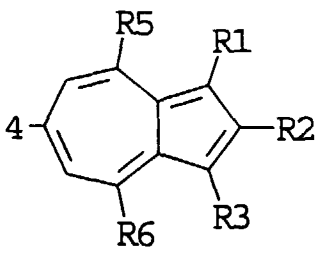

I

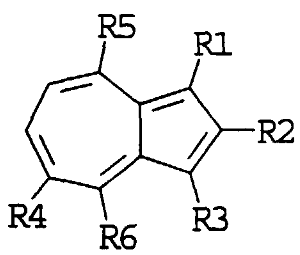

II

wobei

$R_1$, $R_2$ und $R_3$ einzeln oder unabhängig voneinander
Wasserstoff, Hydroxy-, Amino-, Mercapto-, ein gerader oder verzweigter, gesättigter oder ungesättigter aliphatischer Rest mit 1-15 Kohlenstoffatomen, der ein oder mehrfach substituiert sein kann, eine Alkoxy-, Alkylamino-, Di-Alkylamino-, Alkylmercapto-, Alkylsulfinyl-, Alkylsufonyl-, Alkenyl-, Alkinyl-, Alkenyloxy-, Alkenylmercapto-, Alkinyloxy-, Alkinylmercapto-, Alkylcarbonylamino-, Di-N-Alkylcarbonylamino-, Alkylaminocarbonyl-, Formyl-, Alkylcarbonyl-, Carboxyl-, Alkoxycarbonyl-, Alkenyloxycarbonyl-, Alkinyloxycarbonyl-, Carboxyalkyl-, Alkyloxycarbonylalkyl, Alkenyloxycarbonylalkyl-, Alkinyloxycarbonylalkyl-, Nitro-, Cyano-, Halogen-, Trifluormethyl-, Azidogruppe, Phenyl, oder Phenylcarbonyl, das gegebenenfalls ein oder mehrfach unabhängig voneinander substituiert sein kann, einen carbocyclischen mono-, bi- oder tricyclischen Rest mit 7-15 C-Atomen oder ein heterocyclisches mono-, bi- oder tricyclisches Ringsystem bedeuten,

$R_4$ im Fall von Verbindungen der allgemeinen Formel I
$(CH_2)_nCH_2R_7$, $(CH_2)_nCHR_7R_8$, $CH(OR_8)R_7$, $CH(SR_8)R_7$, $NR_9R_{10}$, $OR_{10}$, $SR_{10}$, $C(O)R_{10}$,

im Fall von Verbindungen der allgemeinen Formel II
$R_7$, $(CH_2)_nCH_2R_7$, $(CH_2)_nCHR_7R_8$, $CH(OR_8)R_7$, $CH(SR_8)R_7$, $NR_9R_{10}$, $OR_{10}$, $SR_{10}$, $C(O)R_{10}$,

n die Zahl 0 oder 1

$R_5$ und $R_6$ einzeln und unabhängig voneinander
Wasserstoff, ein gerader oder verzweigter, gesättigter oder ungesättigter aliphatischer Rest mit 1-15 Kohlenstoffatomen, der ein oder mehrfach substituiert sein kann, eine Alkoxy-, Alkylamino-, Di- Alkylamino-, Alkylmercapto-, Alkylsulfinyl-, Alkylsufonyl-, Alkenyl-, Alkinyl-, Alkenyloxy-, Alkenylmercapto-, Alkinyloxy-, Alkinylmercapto-, Alkylcarbonylamino-, Di- -Alkylcarbonylamino-, Alkylaminocarbonyl-, Formyl-, Alkylcarbonyl-, Carboxyl-, Alkoxycarbonyl-, Alkenyloxycarbonyl-, Alkinyloxycarbonyl-, Carboxyalkyl-, Alkyloxycarbonylalkyl, Alkenyloxycarbonylalkyl-, Alkinyloxycarbonylalkyl-, Nitro-, Cyano-, Halogen-, Trifluormethyl-, Azidogruppe, Phenyl, oder Phenylcarbonyl, das gegebenenfalls ein oder mehrfach unabhängig voneinander substituiert sein kann, einen carbocyclischen mono-, bi- oder tricyclischen Rest mit 7-15 C-Atomen oder ein heterocyclisches mono-, bi- oder tricyclisches Ringsystem,

$R_7$,
Carboxyl-, Carboxoureido-, Alkoxycarbonyl-, Alkenyloxycarbonyl-, Alkinyloxycarbonyl-, Aminocarbonyl-, Alkylaminocarbonyl-, Alkenyloxycarbonylalkyl-, Alkinyloxycarbonylalkyl-, *N*-Hydroxyaminocarbonyl-, *N*-Alkoxyaminocarbonyl-, *N*-Alkyl-*N*-Hydroxyaminocarbonyl-, *N*-Alkyl-*N*-Alkoxyaminocarbonyl-, Mercapto-, Alkylmercapto-, Phenylmercapto-, Pyridylmercapto, Halogen-, Dihydroxyboryl-, Di-Alkoxyboryl-, Sulfonsäure-, Phosphonsäure-, Barbitursäure-,

$R_8$

Wasserstoff, Alkyl-, Alkenyl-, Alkinyl-, Phenyl-, Pyridyl-, Thiophenyl-, Alkoxycarbonyl-, und

$R_9$
Wasserstoff, Benzyl, Phenyl, Alkyl bedeutet, und

$R_{10}$
Wasserstoff, Carboxyl-, Alkoxycarbonyl-, Alkenyloxycarbonyl-, Alkinyloxycarbonyl-, Aminocarbonyl-, Alkylaminocarbonyl-, Alkenyloxycarbonylalkyl-, Alkinyloxycarbonylalkyl-, *N*-Hydroxyaminocarbonyl-, *N*-Alkoxyaminocarbonyl-, *N*-Alkyl-*N*-Hydroxyaminocarbonyl-, *N*-Alkyl-*N*-Alkoxyaminocarbonyl- bedeutet

sowie deren Tautomere, Enantiomere, Diastereomere, Racemate und physiologisch verträglichen Salze oder Ester und Substanzen, die *in vivo* zu Verbindungen der Formel I bzw. II hydrolysiert oder metabolisiert werden.
Die Erfindung betrifft auch Verfahren zur Herstellung der obigen Verbindungen, Arzneimittel, die solche Verbindungen enthalten sowie die Verwendung dieser Verbindungen bei der Herstellung von Arzneimitteln.

In Verbindungen der allgemeinen Formel I und II muß mindestens einer der Reste $R_1$, $R_3$, $R_5$ oder $R_6$ ungleich Wasserstoff sein.

Einige Verbindungen der allgemeinen Formel I, bei denen $R_1$, $R_2$ und $R_7$ Ethoxycarbonyl bedeuten, sind in *Chem. Ber.* **1986**, 2272 bzw. 2956 beschrieben. Eine weitere Verbindung der allgemeinen Formel I, bei der $R_1$ und $R_2$ Ethoxycarbonyl- und $R_4$ Dimethylaminocarbonylthio- bedeuten, findet sich in *Tetrahedron Lett.* **1989**, 6345.

Eine Verbindung der allgemeinen Formel II, bei der $R_1$ und $R_3$ *tert*. Butyl- und der Rest $R_4$ Sulfonyl- bedeuten, ist beschrieben in *Rec. Trav. Chim. Pays-Bas* **1985,** 25. Azulen-1,7-dicarbonsäure-7-ethylester-1-methylester und Azulen-1,5-dicarbonsäure-5-ethylester-1-methylester werden in *Justus Liebigs Ann. Chem.* **1957**, 145 erwähnt. Für keine dieser Substanzen sind pharmakologische Wirkungen beschrieben.

Überraschenderweise sind Verbindungen der allgemeinen Formel I oder II Inhibitoren von Metalloproteinasen.
Metalloproteasen spielen in vielen physiologischen und pathophysiologischen Prozessen eine große Rolle.
Beispiele dafür sind das Angiotensin Converting Enzyme (ACE) und die neutrale Endopeptidase (NEP, EC 3.4.24.11), die am Metabolismus einer Reihe von blutdruckregulierenden Peptiden (z. B. Angiotensin I und ANF (atrial natriuretic factor)) beteiligt sind. ACE katalysiert die Spaltung des Angiotensin I zu dem blutdrucksteigernden Angiotensin II. NEP ist für den Abbau des vasodilatierenden Peptids ANF veranwortlich. Das Endothelin Converting Enzyme (ECE) spaltet das endogene, inaktive big-Endothelin zu dem effektiven Vasokonstriktor Endothelin-1, einem aus 21 Aminosäuren bestehenden Peptid. Die Inhibierung dieser Enzyme hat eine große therapeutische Bedeutung zur Behandlung des Bluthochdrucks, der Herzinsuffizienz, des Nierenversagens und des Schlaganfalls. BMP-1 (bone morphogenic factor 1) wurde als Metalloprotease erkannt, die bei der Umwandlung von Procollagen in fibrilläres Collagen eine Rolle spielt. Inhibitoren dieses Enzyms sind für die Behandlung von Fibrosen und sklerotischen Prozessen geeignet und können auch die Narbenbildung bei der Wundheilung günstig beeinflussen. (*Proc. Natl. Acad. Sci. USA* **1996**, *93*, 5127; *Science* **1996**, *271*, 360).

Während Inhibitoren des ACE bereits therapeutisch angewandt werden (z. B. Captopril, Enalapril, (*Exp.Opin. Ther. Pat.* **1996,** *6*, 1147), sind für die Metalloproteasen wie NEP, ECE bisher keine klinisch verwendbaren Wirkstoffe bekannt, die frei von unerwünschten Nebenwirkungen und oral verfügbar sind. (Literaturübersichten: NEP: *Pharmacol. Rev.* **1993**, *45*, 87; ECE: *Bioorg. Med. Chem. Lett.* **1996**, *6*, 2317, zit. Lit. zu Inhibitoren vom Phosporamidontyp. Für das BMP-1 sind bisher noch keine niedermolekularen Inhibitoren bekannt.

Eine andere Gruppe der Metalloproteasen ist die der Matrixmetalloproteasen (MMPs). In normalem Gewebe besteht ein Gleichgewicht zwischen Auf- und Abbau der extrazellulären Matrix. Die extrazelluläre Matrix wird von wenigstens drei Gruppen Proteasen, nämlich den Collagenasen, Gelatinasen und den Stromelysinen abgebaut.
Normalerweise sorgen spezifische Inhibitoren dieser Enzyme, wie z.B. $\alpha_2$-Makroglobulin und TIMP (tissue inhibitor of metalloproteases) dafür, daß kein übermäßiger Abbau der extrazellulären Matrix stattfindet. Eine verwandte Gruppe von Proteasen ist die der Adamalysine, mit dem TNF-$\alpha$ converting enzyme (TACE) (Moss et al., *Nature* **1996**, *385*, 733).
Wenigstens 11 verschiedene und doch sehr homologe Matrixmetalloproteinasen wurden charakterisiert, darunter die interstitial fibroblast collagenase (MMP-1, HFC), die neutrophile Collagenase (MMP-8, HNC), zwei Gelatinasen, Stromelysine (z.B. HSL-1) und Matrilysin (Birkedal-Hansen, H., Moore, W.G.I., Bodden, M.K. Windsor, L.J., Birkedahl-Hansen,B., DeCarlo, A., Engler, J.A., *Critical Rev. Oral Biol. Med.* **1993**, *4*, 197-250). Diese Proteinasen teilen eine Reihe struktureller und funktionaler Eigenschaften, unterscheiden sich allerdings in ihrer Substratspezifität. Nur HNC und HFC spalten natives triple-helikales Collagen der Typen I, II und III. Dabei entstehen Fragmente von 3/4 und 1/4 der ursprünglichen Länge. Durch diesen Abbau wird der Schmelzpunkt des Collagens erniedrigt. Anschließend kann es durch andere matrixabbauende Enzyme angegriffen werden.

Der unkontrollierte exzessive Abbau dieser Matrizes ist für viele pathologische Erscheinungsbilder, wie z.B. rheumatoide Arthritis, Osteoarthritis, Multiple Sklerose, Tumor Metastasierung, Comea Ulzerationen, Inflammatorische Prozesse und verschiedene Erkrankungen der Knochen und Zähne, typisch.
Die Pathogenese dieser Krankheiten sollte durch die Gabe von Metalloproteinaseinhibitoren positiv beeinflußt werden. In der Literatur finden sich inzwischen einige solche Verbindungen (eine Übersicht findet sich z.B. bei Nigel R. A. Beeley et al., Curr. Opin. Ther. Patents 4 (1), 7 (**1994**)). Dabei handelt es sich vor allem um Peptide mit einem Hydroxamsäure, Thiol- oder Phosphinrest als zinkbindende Gruppe (unter anderem z.B. WO-A-9209563 von Glycomed, EP-A-497192 von Hoffmann-LaRoche, WO-A-489577 von Celltech, EP-A-320118 von Beecham, US-A-4595700 von Searle).

Tumor Nekrose Faktor $\alpha$ (TNF$\alpha$) ist ein proinflammatorisches Zytokin, das bei einer Vielzahl von Erkrankungen pathogenetische Bedeutung besitzt. Klinisch wurde in einer multizentrischen, randomisierten Doppel-Blind-Studie von Elliot et al. in Lancet 344, 1105 - 1110 (**1994**) gezeigt, daß ein neutralisierender Antikörper gegen TNF$\alpha$ eine schnelle und ausgeprägte Besserung der Krankheitssymptome bei Patienten mit rheumatoiden Arthritis bewirken. Mittlerweile wurden von van Dullemen et al. in Gastroenterology 109, 129 - 135 (**1995**) klinische Daten publiziert, die eine therapeutische Wirkung solcher Antikörper bei Patienten mit Morbus Crohn belegen. Weiterhin wurde tierexperimentell gezeigt, daß Rolipram, das ebenfalls die Synthese von TNF$\alpha$ blockiert, in Tiermodellen für die Multiple Sklerose sehr gut wirksam ist. Thalidomid, eine weitere TNF$\alpha$-hemmende Substanz, wird klinisch zur Behandlung von chronischer Graft versus Host-Erkrankung, bei der Behandlung von Lepra nodulare, und von Patienten mit Lupus erythematodes eingesetzt. Zudem wurde für diese Substanz gezeigt, daß sie die Proliferation von HIV unterdrückt.

Bei folgenden Krankheitsbildern scheint TNF$\alpha$ von direkter pathogenetischer Bedeutung zu sein:
Degenerative Gelenkserkrankungen, rheumatoide Arthritis, Entzündung, Allergie, ARDS, Asthma, Herzinfarkt, Chronische Herzinsuffizienz, HIV-Infektion, Morbus Crohn, Ulzerative Colitis, Psoriasis, Dermatitis, Actinokeratose, Vaskulitiden, septischer Schock, Transplantatabstoßung, Multiple Skerose, Ulzera, Diabetes, chronische Graft versus Host-Erkrankung, Lepra und andere Infektionskrankheiten, Lupus Erythematodes, Paradontose und bei anderen Erkrankungen.

Der klinische Einsatz von monoklonalen Anti- TNF$\alpha$-Antikörpern kann nur parenteral erfolgen. Das Arzneimittel ist teuer herzustellen und erfordert eine aufwendige Vertriebslogistik (Kühlkette, Lagerung, Verfallzeit, etc.). Zudem wird bei 50 % der Patienten, die zwischen 2 und 4 Injektionen bekommen haben, das Auftreten neutralisierender HACAs (Human anti-chimeric antibodies) festgestellt. Dies führt dazu, daß die beschwerdefreien Phasen immer kürzer werden. Die Entwickiung von Rolipram als Anti-TNF$\alpha$-Therapieprinzip ist durch dessen emetische Wirkung beeinträchtigt. Die teratogenen Nebenwirkungen von Thalidomid und die schwache TNF$\alpha$-Blockade lassen eine klinische Entwicklung dieser Substanz ebenfalls schwierig erscheinen.
Überraschenderweise sind Verbindungen der allgemeinen Formel I oder II Inhibitoren von Metalloproteinasen, insbesondere von TNF$\alpha$.

Die Verbindungen der allgemeinen Formeln I und II, die Gegenstand der Erfindung sind, sind im folgenden näher beschrieben. Die Erfindung betrifft Substanzen der allgemeinen Formeln I und II,

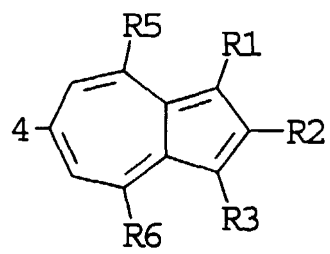

I

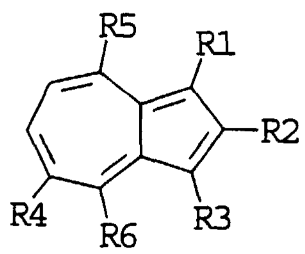

II

wobei

$R_1$, $R_2$ und $R_3$ einzeln oder unabhängig voneinander
Wasserstoff, Hydroxy-, Amino-, Mercapto-, Thio-, ein gerader oder verzweigter, gesättigter oder ungesättigter aliphatischer Rest mit 1 - 15 Koblenstoffatomen, der ein oder mehrfach Hydroxy-, Amino-, Mercapto-, Thio-, $C_1$-$C_6$-Alkoxy-, $C_1$-$C_6$-Alkylamino-, Di-$C_1$-$C_6$-Alkylamino-, $C_1$-$C_6$-Alkylmercapto-, $C_1$-$C_6$-Alkylsulfinyl-, $C_1$-$C_6$-Alkylsulfonyl-, $C_2$-$C_6$-Alkenyl-, $C_2$-$C_6$-Alkinyl-, $C_2$-$C_6$-Alkenyloxy-, $C_2$-$C_6$-Alkenylmercapto-, $C_2$-$C_6$-Alkinyloxy-, $C_2$-$C_6$-Alkinyl-

mercapto-, $C_1$-$C_6$-Alkylcarbonylamino-, $C_1$-$C_6$-Alkylaminocarbonyl-, Formyl-, $C_1$-$C_6$-Alkylcarbonyl-, Carboxyl-, $C_1$-$C_6$-Alkoxycarbonyl-, $C_1$-$C_6$-Alkenyloxycarbonyl-, $C_1$-$C_6$-Alkinyloxycarbonyl, Carboxy-$C_1$-$C_6$-alkyl-, $C_1$-$C_6$-Alkyloxycarbonyl-$C_1$-$C_6$-alkyl-, $C_2$-$C_6$-Alkenyloxycarbonyl-$C_1$-$C_6$-alkyl-, $C_2$-$C_6$-Alkinyloxycarbonyl-$C_1$-$C_6$-alkyl-, Benzyloxy-, Phenylmercapto-, Phenyloxy-, Nitro-, Cyano-, Halogen-, Trifluormethyl-, Azido-, Formylamino-, oder Phenyl- substituiert sein kann,

eine $C_1$-$C_6$-Alkoxy-, $C_1$-$C_6$-Alkylamino-, Di-$C_1$-$C_6$-Alkylamino-, $C_1$-$C_6$-Alkylmercapto-, $C_1$-$C_6$-Alkylsulfinyl-, $C_1$-$C_6$-Alkylsufonyl-, $C_2$-$C_6$-Alkenyl-, $C_2$-$C_6$-Alkinyl-, $C_2$-$C_6$-Alkenyloxy-, $C_2$-$C_6$-Alkenylmercapto-, $C_2$-$C_6$-Alkinyloxy-, $C_2$-$C_6$-Alkinylmercapto-, $C_1$-$C_6$-Alkylcarbonylamino-, Di-$C_1$-$C_6$-Alkylcarbonylamino-, $C_1$-$C_6$-Alkylaminocarbonyl-, Formyl-, $C_1$-$C_6$-Alkylcarbonyl-, Carboxyl-, $C_1$-$C_6$-Alkoxycarbonyl-, $C_1$-$C_6$-Alkenyloxycarbonyl-, $C_1$-$C_6$-Alkinyloxycarbonyl-, Carboxy-$C_1$-$C_6$-alkyl-, $C_1$-$C_6$-Alkyloxycarbonyl-$C_1$-$C_6$-alkyl-, $C_2$-$C_6$-Alkenyloxycarbonyl-$C_1$-$C_6$-alkyl-, $C_2$-$C_6$-Alkinyloxycarbonyl-$C_1$-$C_6$-alkyl-, Nitro-, Cyano-, Halogen-, Trifluormethyl-, Azidogruppe,

Phenyl, oder Phenylcarbonyl, das gegebenenfalls ein oder mehrfach unabhängig voneinander durch Hydroxy-, Amino-, Mercapto-, $C_1$-$C_6$-Alkoxy-, $C_1$-$C_6$-Alkylamino-, Di-$C_1$-$C_6$-Alkylamino-, $C_1$-$C_6$-Alkylmercapto-, $C_1$-$C_6$-Alkylsulfinyl-, $C_1$-$C_6$-Alkylsufonyl-, $C_2$-$C_6$-Alkenyl-, $C_2$-$C_6$-Alkinyl-, $C_2$-$C_6$-Alkenyloxy-, $C_2$-$C_6$-Alkenylmercapto-, $C_2$-$C_6$-Alkinyloxy-, $C_2$-$C_6$-Alkinylmercapto-, $C_1$-$C_6$-Alkylcarbonylamino-, $C_1$-$C_6$-Alkylaminocarbonyl-, Formyl-, $C_1$-$C_6$-Alkylcarbonyl-, Carboxyl-, $C_1$-$C_6$-Alkoxycarbonyl-, $C_1$-$C_6$-Alkenyloxycarbonyl-, $C_1$-$C_6$-Alkinyloxycarbonyl-, Carboxy-$C_1$-$C_6$-alkyl-, $C_1$-$C_6$-Alkyloxycarbonyl-$C_1$-$C_6$-alkyl-, $C_2$-$C_6$-Alkenyloxycarbonyl-$C_1$-$C_6$-alkyl-, $C_2$-$C_6$-Alkinyloxycarbonyl-$C_1$-$C_6$-alkyl-, Benzyloxy-, Phenylmercapto-, Phenyloxy-, Nitro-, Cyano-, Halogen-, Trifluormethyl-, Azido-, Formylamino-, Carboxy- oder Phenyl- substituiert sein kann,

einen carbocyclischen mono-, bi- oder tricyclischen Rest mit 7-15 C-Atomen oder ein heterocyclisches mono-, bi- oder tricyclisches Ringsystem darstellen kann, wobei die ungesättigten bzw. aromatischen Carbo- und Heterocyclen partiell oder vollständig hydriert sein können.

$R_4$ bedeutet im Fall von Verbindungen der allgemeinen Formel I
$(CH_2)_nCH_2R_7$, $(CH_2)_nCHR_7R_8$, $CH(OR_8)R_7$, $CH(SR_8)R_7$, $NR_9R_{10}$, $OR_{10}$, $SR_{10}$ oder $C(O)R_{10}$,

im Fall von Verbindungen der allgemeinen Formel II
$R_7$, $(CH_2)_nCH_2R_7$, $(CH_2)_nCHR_7R_8$, $CH(OR_8)R_7$, $CH(SR_8)R_7$, $NR_9R_{10}$, $OR_{10}$, $SR_{10}$ oder $C(O)R_{10}$.

n die Zahl 0 oder 1

$R_5$ und $R_6$ bedeuten einzeln und unabhängig voneinander
Wasserstoff, einen geraden oder verzweigten, gesättigten oder ungesättigten aliphatischen Rest mit 1-15 Kohlenstoffatomen, der ein oder mehrfach Hydroxy-, Amino-, Mercapto-, $C_1$-$C_6$-Alkoxy-, $C_1$-$C_6$-Alkylamino-, Di-$C_1$-$C_6$-Alkylamino-, $C_1$-$C_6$-Alkylmercapto-, $C_1$-$C_6$-Alkylsulfinyl-, $C_1$-$C_6$-Alkylsulfonyl-, $C_2$-$C_6$-Alkenyl-, $C_2$-$C_6$-Alkinyl-, $C_2$-$C_6$-Alkenyloxy-, $C_2$-$C_6$-Alkenylmercapto-, $C_2$-$C_6$-Alkinyloxy-, $C_2$-$C_6$-Alkinylmercapto-, $C_1$-$C_6$-Alkylcarbonylamino-, $C_1$-$C_6$-Alkylaminocarbonyl-, Formyl-, $C_1$-$C_6$-Alkylcarbonyl-, Carboxyl-, $C_1$-$C_6$-Alkoxycarbonyl-, $C_1$-$C_6$-Alkenyloxycarbonyl-, $C_1$-$C_6$-Alkinyloxycarbonyl-, Carboxy-$C_1$-$C_6$-alkyl-, $C_1$-$C_6$-Alkyloxycarbony-$C_1$-$C_6$-alkyl-, $C_2$-$C_6$-Alkenyloxycarbonyl-$C_1$-$C_6$-alkyl-, $C_2$-$C_6$-Alkinyloxycarbonyl-$C_1$-$C_6$-alkyl-, Benzyloxy-, Phenylmercapto-, Phenyloxy-, Nitro-, Cyano-, Halogen-, Trifluormethyl-, Azido-, Formylamino-, oder Phenylsubstituiert sein kann,

eine $C_1$-$C_6$-Alkoxy-, $C_1$-$C_6$-Alkylamino-, Di-$C_1$-$C_6$-Alkylamino-, $C_1$-$C_6$-Alkylmercapto-, $C_1$-$C_6$-Alkylsulfinyl-, $C_1$-$C_6$-Alkylsufonyl-, $C_2$-$C_6$-Alkenyl-, $C_2$-$C_6$-Alkinyl-, $C_2$-$C_6$-Alkenyloxy-, $C_2$-$C_6$-Alkenylmercapto-, $C_2$-$C_6$-Alkinyloxy-, $C_2$-$C_6$-Alkinylmercapto-, $C_1$-$C_6$-Alkylcarbonylamino-, Di-$C_1$-$C_6$-Alkylcarbonylamino-, $C_1$-$C_6$-Alkylaminocarbonyl-, Formyl-, $C_1$-$C_6$-Alkylcarbonyl-, Carboxyl-, $C_1$-$C_6$-Alkoxycarbonyl-, $C_1$-$C_6$-Alkenyloxycarbonyl-, $C_1$-$C_6$-Alkinyloxycarbonyl-, Carboxy-$C_1$-$C_6$-alkyl-, $C_1$-$C_6$-Alkyloxycarbonyl-$C_1$-$C_6$-alkyl-, $C_2$-$C_6$-Alkenyloxycarbonyl-$C_1$-$C_6$-alkyl-, $C_2$-$C_6$-Alkinyloxycarbonyl-$C_1$-$C_6$-alkylgruppe,

Phenyl, das gegebenenfalls ein oder mehrfach unabhängig voneinander durch Hydroxy-, Amino-, Mercapto-, $C_1$-$C_6$-Alkoxy-, $C_1$-$C_6$-Alkylamino-, Di-$C_1$-$C_6$-Alkylamino-, $C_1$-$C_6$-Alkylmercapto-, $C_1$-$C_6$-Alkylsulfinyl-, $C_1$-$C_6$-Alkylsufonyl-, $C_2$-$C_6$-Alkenyl-, $C_2$-$C_6$-Alkinyl-, $C_2$-$C_6$-Alkenyloxy-, $C_2$-$C_6$-Alkenylmercapto-, $C_2$-$C_6$-Alkinyloxy-, $C_2$-$C_6$-Alkinylmercapto-, $C_1$-$C_6$-Alkylcarbonylamino-, $C_1$-$C_6$-Alkylaminocarbonyl-, Formyl-, $C_1$-$C_6$-Alkylcarbonyl-, Carboxyl-, $C_1$-$C_6$-Alkoxycarbonyl-, $C_1$-$C_6$-Alkenyloxycarbonyl-, $C_1$-$C_6$-Alkinyloxycarbonyl-, Carboxy-$C_1$-$C_6$-alkyl-, $C_1$-$C_6$-Alkyloxycarbonyl-$C_1$-$C_6$-alkyl-, $C_2$-$C_6$-Alkenyloxycarbonyl-$C_1$-$C_6$-alkyl-, $C_2$-$C_6$-Alkinyloxycarbonyl-$C_1$-$C_6$-alkyl-, Benzyloxy-, Phenylmercapto-, Phenyloxy-, Nitro-, Cyano-, Halogen-, Trifluormethyl-, Azido-, Formylamino-, Carboxy- oder Phenyl- substituiert sein kann,

einen carbocyclischen mono-, bi- oder tricyclischen Rest mit 7-15 C-Atomen oder ein heterocyclisches mono-, bi- oder tricyclisches Ringsystem darstellen kann, wobei die ungesättigten bzw. aromatischen Carbo- und Heterocyclen partiell oder vollständig hydriert sein können.

$R_7$ symbolisiert einen
Carboxyl-, Carboxoureido-, $C_1$-$C_6$-Alkoxycarbonyl-, $C_2$-$C_6$-Alkenyloxycarbonyl-, $C_2$-$C_6$-Alkinyloxycarbonyl-, Aminocarbonyl-, $C_1$-$C_6$-Alkylaminocarbonyl-, $C_2$-$C_6$-Alkenyloxycarbonyl-$C_1$-$C_6$-alkyl-, $C_2$-$C_6$-Alkinyloxycarbonyl-$C_1$-$C_6$-alkyl-, *N*-Hydroxyaminocarbonyl-, *N*-$C_1$-$C_6$-Alkoxyaminocarbonyl-, *N*-$C_1$-$C_6$-Alkyl-*N*-Hydroxyaminocarbonyl-, *N*-$C_1$-$C_6$-Alkyl-*N*-$C_1$-$C_6$-Alkoxyaminocarbonyl-, Mercapto-, $C_1$-$C_6$-Alkylmercapto-, Phenylmercapto-, Pyridylmercapto, Halogen-, Dihydroxyboryl-, Di-$C_1$-$C_6$-Alkoxyboryl-, Sulfonsäure-, Phosphinsäure-, Phosphonsäure-, oder Barbitursäurerest.

$R_8$ stellt
Wasserstoff, oder einen $C_1$-$C_6$-Alkyl-, $C_2$-$C_6$-Alkenyl-, $C_2$-$C_6$-Alkinyl-, $C_1$-$C_6$-Alkoxycarbonyl-, Phenyl-, Pyridyl-, Thiophenylrest dar.

$R_9$ bedeutet Wasserstoff, Benzyl, Phenyl, oder $C_1$-$C_6$ Alkyl.

$R_{10}$ bedeutet
Wasserstoff, Carboxyl-, $C_1$-$C_6$-Alkoxycarbonyl-, $C_2$-$C_6$-Alkenyloxycarbonyl-, $C_2$-$C_6$-Alkinyloxycarbonyl-, Aminocarbonyl-, $C_1$-$C_6$-Alkylaminocarbonyl-, $C_2$-$C_6$-Alkenyloxycarbonyl-$C_1$-$C_6$-alkyl-, $C_2$-$C_6$-Alkinyloxycarbonyl-$C_1$-$C_6$-alkyl-, *N*-Hydroxyaminocarbonyl-, *N*-$C_1$-$C_6$-Alkoxyaminocarbonyl-, *N*-$C_1$-$C_6$-Alkyl-*N*-Hydroxyaminocarbonyl-, *N*-$C_1$-$C_6$-Alkyl-*N*-$C_1$-$C_6$-Alkoxyaminocarbonyl-,

Ein aliphatischer Rest bedeutet einen geradkettigen, verzweigten oder cyclischen Alkyl-, Alkenyl- oder Alkinylrest mit 1-15, vorzugsweise 1-10 Kohlenstoffatomen, wie z. B. der Methyl-, Ethyl-, Propyl-, Isopropyl-, Butyl-, *sec*-Butyl-, *tert*-Butyl-, Pentyl-, Hexyl-, Heptyl-, Oktyl-, Nonyl-, Decyl-, Cyclopropyl-, Cyclobutyl-, Cyclohexylrest. Ungesättigte Reste sind z. B. der Vinyl-, Allyl-, Dimethylallyl-, Butenyl-, Isobutenyl-, Pentenyl-, Ethinyl- oder Propinylrest.

$C_1$-$C_6$-Alkylreste in $C_1$-$C_6$-Alkyl, $C_1$-$C_6$-Alkoxy-, $C_1$-$C_6$-Alkylamino-, Di-$C_1$-$C_6$-Alkylamino-, $C_1$-$C_6$-Alkylmercapto-, $C_1$-$C_6$-Alkylsulfinyl-, $C_1$-$C_6$-Alkylsufonyl-, $C_1$-$C_6$-Alkylcarbonylamino-, $C_1$-$C_6$-Alkylaminocarbonyl-, $C_1$-$C_6$-Alkoxycarbonyl-, $C_1$-$C_6$-Alkoxycarbonyl, Carboxy-$C_1$-$C_6$-alkyl-, $C_1$-$C_6$-Alkyloxycarbonyl-$C_1$-$C_6$-alkyl-, $C_2$-$C_6$-Alkenyloxycarbonyl-$C_1$-$C_6$-alkyl-, $C_2$-$C_6$-Alkinyloxycarbonyl-$C_1$-$C_6$-alkyl-, bedeuten geradkettige, verzweigte oder cyclische Reste. Bevorzugt sind die Methyl-, Ethyl-, Propyl-, Isopropyl-, Butyl-, *sec*- oder *tert*-Butyl-, Pentyl-, Hexyl-, Cyclopropyl-, Cyclobutyl-, Cyclohexyl-, Methoxy-, Ethoxy-, Propyloxy-, Isopropyloxy-, Butyloxy-, *sec*-Butyl-, *tert*-Butyloxy-, Methoxycarbonyl-, Ethoxycarbonyl-, Propyloxycarbonyl-, Butyloxycarbonyl-, Carboxymethyl-, Carboxyethyl-, Cärboxypropyl-, Methoxycarbonylethyl-, Ethoxycarbonylethyl-, Methoxycarbonylpropyl-, Ethoxycarbonylpropyl-, Carboxymethoxy-, Carboxyethoxy-, Carboxypropyloxy-, Methoxycarbonylmethoxy-, Ethoxycarbonylethoxy-, Propoxycarbonylmethoxy-, Methoxycarbonylethoxy-, Ethoxycarbonylethoxy-, Aminomethyl-, Aminoethyl-, Aminopropyl-, Methylmercapto-, Ethylmercapto-, Propylmercaptogruppe.

$C_2$-$C_6$-Alkenyl- und $C_2$-$C_6$-Alkinylreste in $C_2$-$C_6$-Alkenyl, $C_2$-$C_6$-Alkinyl, $C_2$-$C_6$-Alkenyloxy, $C_2$-$C_6$-Alkenylmercapto, $C_2$-$C_6$-Alkinyloxy, $C_2$-$C_6$-Alkinylmercapto-, $C_2$-$C_6$-Alkenyloxycarbonyl-, $C_2$-$C_6$-Alkinyloxycarbonyl, $C_2$-$C_6$-Alkenyloxycarbonyl-$C_1$-$C_6$-alkyl-, $C_2$-$C_6$-Alkinyloxycarbonyl-$C_1$-$C_6$-alkyl-, bedeuten geradkettige, verzweigte oder cyclische Reste. Bevorzugt sind die Vinyl-, Propenyl-, Butenyl-, Pentenyl-, Hexenyl-, Ethinyl-, Propargyl-, Vinyloxy-, Allyloxy-, Propargyloxy-, Allyloxycarbonyl-, Propargyloxycarbonyl-, Allyloxycarbonylmethyl-, Allyloxycarbonylethyl-, Allyloxycarbonylpropyl-, Propargyloxycarbonylmethyl-, Propargyloxycarbonylethyl- und die Propargyloxycarbonylpropylgruppe.

Ein carbocyclischer Ring mit 7-15 C-Atomen kann mono-, bi- oder tricyclisch sein und pro Ring jeweils 5-7 C-Atome aufweisen. Dieser Ring kann aromatisch oder ganz oder teilweise gesättigt sein. Bevorzugt sind der Naphtyl-, Anthracenyl-, Phenanthrenyl-, Fluorenyl-, Indenyl-, Acenaphtylenyl-, Norbornyl-, Adamantylring oder eine $C_3$-$C_7$-Cycloalkyl oder $C_5$-$C_8$-Cycloalkenylgruppe. Der carbocyclische Ring kann darüberhinaus 1-3 fach substituiert sein, wobei die Substituenten unabhängig voneinander ein gerader oder verzweigter, gesättigter oder ungesättigter aliphatischer Rest mit 1-9 Kohlenstoffatomen, eine Hydroxy-, Amino-, Mercapto-, $C_1$-$C_6$-Alkoxy-, $C_1$-$C_6$-Alkylamino-, Di-$C_1$-$C_6$-Alkylamino-, $C_1$-$C_6$-Alkylmercapto-, $C_1$-$C_6$-Alkylsulfinyl-, $C_1$-$C_6$-Alkylsufonyl-, $C_2$-$C_6$-Alkenyl-, $C_2$-$C_6$-Alkinyl-, $C_2$-$C_6$-Alkenyloxy-, $C_2$-$C_6$-Alkenylmercapto-, $C_2$-$C_6$-Alkinyloxy-, $C_2$-$C_6$-Alkinylmercapto-, $C_1$-$C_6$-Alkylcarbonylamino-, $C_1$-$C_6$-Alkylaminocarbonyl-, Carbonyl-, $C_1$-$C_6$-Alkylcarbonyl-, Carboxyl-, $C_1$-$C_6$-Alkoxycarbonyl-, $C_1$-$C_6$-Alkenyloxycarbonyl-, $C_1$-$C_6$-Alkinyloxycarbonyl-, Carboxy-$C_1$-$C_6$-alkyl-, $C_1$-$C_6$-Alkyloxycarbonyl-$C_1$-$C_6$-alkyl-, $C_2$-$C_6$-Alkenyloxycarbonyl-$C_1$-$C_6$-alkyl-, $C_2$-$C_6$-Alkinyloxycarbonyl-$C_1$-$C_6$-alkyl-, Benzyloxy-, Phenylmercapto-, Phenyloxy-, Nitro-, Cyano-, Halogen-,

Trifluormethyl-, Azido-, Formylamino-, oder Phenyl- sein können.

Unter einem heterocyclischen mono-, bi- oder tricyclischen Ringsystem versteht man ein gesättigtes oder ungesättigtes Ringsystem mit 5 bis 7 Ringgliedern, welches 1-3 gleiche oder verschiedene Heteroatome wie Stickstoff, Sauerstoff oder Schwefel enthält, wie z.B. das Pyridin-, Pyrimidin-, Pyridazin-, Pyrazin-, Triazin-, Pyrrol-, Pyrazol-, Imidazol-, Triazol-, Thiazol-, Oxazol-, Isoxazol-, Oxadiazol-, Furazan-, Furan-, Thiophen-, Indol-, Chinolin-, Isochinolin-, Cumaron-, Thionaphten-, Benzoxazol-, Benzthiazol-, Indazol-, Benzimidazol-, Benztriatol-, Chromen-, Phtalazin-, Chinazolin-, Chinoxalin-, Methylendioxybenzol-, Carbazol-, Acridin-, Phenoxazin-, Phenothiazin-, Phenazin-, oder Purinsystem, wobei die ungesättigten bzw. aromatischen Carbo- und Hetercyclen partiell oder vollständig hydriert sein können. Das heterocyclische System kann darüberhinaus ein oder mehrfach substituiert sein, wobei die Substituenten unabhängig voneinander ein gerader oder verzweigter, gesättigter oder ungesättigter aliphatischer Rest mit 1-9 Kohlenstoffatomen, eine Hydroxy-, Amino-, Mercapto-, $C_1$-$C_6$-Alkoxy-, $C_1$-$C_6$-Alkylamino-, Di-$C_1$-$C_6$-Alkylämlno, $C_1$-$C_6$-Alkylmercapto-, $C_1$-$C_6$-Alkylsulfinyl-, $C_1$-$C_6$-Alkylsufonyl, $C_2$-$C_6$-Alkenyl-, $C_2$-$C_6$-Alkinyl-, $C_2$-$C_6$-Alkenyloxy-, $C_2$-$C_6$-Alkenylmercapto-, $C_2$-$C_6$-Alkinyloxy-, $C_2$-$C_6$-Alkinylmercapto-, $C_1$-$C_6$-Alkylcarbonylamino-, $C_1$-$C_6$-Alkylaminocarbonyl-, Carbonyl-, $C_1$-$C_6$-Alkylcarbonyl-, Carboxyl-, $C_1$-$C_6$-Alkoxycarbonyl-, $C_1$-$C_6$-Alkenyloxycarbonyl-, $C_1$-$C_6$-Alkinyloxycarbonyl-, Carboxy-$C_1$-$C_6$-alkyl-, $C_1$-$C_6$-Alkyloxycarbonyl-$C_1$-$C_6$-alkyl-, $C_2$-$C_6$-Alkenyloxycarbonyl-$C_1$-$C_6$-alkyl-, $C_2$-$C_6$-Alkinyloxycarbonyl-$C_1$-$C_6$-alkyl-, Benzyloxy-, Phenylmercapto-, Phenyloxy-, Nitro-, Cyano-, Halogen-, Trifluormethyl-, Azido-, Formylamino-, oder Phenyl- sein können. Bevorzugt sind Pyrrolidin, Piperidin, Piperazin, Morpholin, Hexahydroazepin, Tetrahydrofuran, Tetrahydropyran, Tetrahydrothiophen, 4,5-Dihydroimidazol, Pyrrol, Imidazol, Pyrazin, Pyrimidin, Pyridazin, 1*H*-Azepin, 3*H*-Azepin, 1,2-Diazepin, 1,4-Diazepin, Furan, Thiophen, Oxazol, Isoxazol, Thiazol, Isothiazol, Pyrazol, Pyrollidinon, Imidazolidinon, Piperidinon, Indol, Purin, Chinolin und Isochinolin.

Insbesondere bevorzugt sind Verbindungen der allgemeinen Formel I bzw. II, in denen $R_1$ und/oder $R_3$ einen Carboxyl- oder einen $C_1$-$C_6$-Alkoxycarbonylrest bedeutet, $R_2$ Amino, $C_1$-$C_6$-Alkylcarbonylamino, Di-$C_1$-$C_6$-Alkylcarbonylamino bedeutet, $R_4$ gleich $(CH_2)_nCH_2R_7$ mit n gleich 0, $R_7$ gleich N-Hydroxyaminocarbonyl, $R_7$ gleich Carboxyl, $R_7$ gleich $C_1$-$C_6$-Alkoxycarbonyl bedeutet, $R_4$ gleich $(CH_2)_nCHR_7R_8$ mit n gleich 0, $R_7$ gleich $C_1$-$C_6$-Alkoxycarbonyl und $R_8$ gleich $C_1$-$C_6$-Alkoxycarbonyl, $R_7$ Carboxoureido und $R_8$ Alkoxycarbonyl bedeutet und $R_5$ und/oder $R_6$ bevorzugt Wasserstoff bedeuten. Bevorzugte Substituenten für $R_1$ und/oder $R_3$ sind der Carboxyl-, Carbonsäureethyl- sowie Carbonsäure*tert*butylester.

Gegenstand der Erfindung sind auch neue Verbindungen der Formel I bzw. II, in denen $R_4$ Wasserstoff oder Halogen bedeutet, wenn $R_1$ und/oder $R_3$ einen Carbonsäure*tert*butylester oder einen Carbonsäure*tert*butylethylester bedeuten und die anderen Substituenten die angegebene Bedeutung haben.

Unter den physiologisch verträglichen Salzen der allgemeinen Formel I bzw. II versteht man beispielsweise Formiate, Acetate, Caproate, Oleate, Lactate oder Salze von Carbonsäuren mit bis zu 18 Koblenstoffatomen oder Salze von Dicarbonsäuren und Tricärbonsäuren wie Citrate, Malonate und Tartrate oder Alkansulfonate mit bis zu 10 Kohlenstoffatomen oder p-Toluolsulfonate oder Salicylate oder Trifluoracetate oder Salze von physiologisch verträglichen Mineralsäuren wie Salzsäure, Bromwasserstoffsäure, Iodwasserstoffsäure, Schwefelsäure, Phosphorsäure. Die Verbindungen der Formel I bzw. II mit freier Carboxylgruppe können auch Salze mit physiologisch verträglichen Basen bilden. Beispiele solcher Salze sind Alkälimetall-, Erdalkalimetall-, Ammonium- und Alkylammoniumsalze, wie das Natrium-, Kalium-, Calcium- oder Tetramethylammoniumsalz.

Zu reinen Enantiomem der Verbindungen der Formel I bzw. II kommt man entweder durch Racematspaltung (über Salzbildung mit optisch aktiven Säuren oder Basen), oder indem man in die Synthese optisch aktive Ausgangsstoffe einsetzt.

Die Verbindungen der Formel I bzw. II können solvatisiert, insbesondere hydratisiert sein. Die Hydratisierung kann im Zuge der Herstellung erfolgen oder allmählich als Folge hygroskopischer Eigenschaften einer zunächst wasserfreien Verbindung der Formel I bzw. II auftreten.

Verbindungen der Formel I bzw. II können in flüssiger, fester oder in Form von Aerosolen oral, enteral, parenteral, topisch, nasal, pulmonal oder rectal in allen üblichen nichttoxischen pharmazeutisch akzeptierten Trägermaterialien, Adjuvantien und Zusätzen verabbreicht werden. Der Begriff parenteral unfaßt dabei subcutane, intravenöse und intramuskuläre Zufuhr oder Infusionen. Orale Applikationsformen sind z.B. in W.A. Ritschel, Die Tablette, 1966, Aulendorf, beschrieben und können z.B. Tabletten, Kapseln, Dragees, Sirupe, Lösungen, Suspensionen, Emulsionen, Elixiere etc. sein, die einen oder mehrere Zusätze aus den folgenden Gruppen enthalten können, wie z.B. Geschmacksstoffe, Süßstoffe, Farbstoffe und Konservierungsmittel. Orale Applikationsformen enthalten den wirksamen Bestandteil zusammen mit nichttoxischen, pharmazeutisch akzeptierten Trägermaterialien, die zur Herstellung von Tabletten, Kapseln, Dragees usw. geeignet sind, wie z.B. Calciumcarbonat, Natriumcarbonat, Lactose, Calciumphosphat oder Natriumphosphat; Stärke, Mannit, Methylcellulose, Talkum, hochdisperse Kieselsäuren, höhermolekulare Fettsäuren (wie Stearinsäure), Erdnußöl, Olivenöl, Paraffin, Miglyol, Gelatine, Agar-Agar, Magnesiumstearat, Bienenwachs, Cetylalkohol, Lecithin, Glycerol, tierische und pflanzliche Fette, feste hochmolekulare Polymere (wie Polyethylenglykole). Tabletten, Kapseln Dragees usw. können mit einem entsprechenden Überzug, wie z.B. Glycerylmonostearat oder Glyceryldistearat versehen werden, so daß unerwünschte Nebenwirkungen im Magen verhindert werden, oder es

durch die verzögerte Absorption im Gastrointestinaltrakt zu einer längeren Wirkungsdauer kommt. Als Injektionsmedium kommen vorzugsweise sterile injizierbare wäßrige oder ölige Lösungen oder Suspensionen zur Anwendung, welche die üblichen Zusätze, wie Stabilisierungsmittel und Lösungsvermittler enthalten. Derartige Zusätze können z.B. Wasser, isotonische Kochsalzlösung, 1,3-Butandiol, Fettsäuren (wie Ölsäure), Mono- und Diglyceride, oder Miglyol sein. Für die rectale Anwendung können alle geeigneten nicht irritierenden Zusätze verwendet werden, die bei normalen Temperaturen fest und bei Rectaltemperatur flüssig sind, wie z. B. Kakaobutter und Polyethylenglykol. Für die Anwendung als Aerosol konunen die pharmazeutisch üblichen Trägermedien zur Anwendung. Für den äußerlichen Gebrauch finden Cremes, Tinkturen, Gele, Losungen oder Suspensionen usw. mit den pharmazeutisch üblichen Zusätzen Anwendung.

Die Dosierung kann von verschiedenen Faktoren, wie Applikationsweise, Spezies, Alter und/oder individuellem Zustand abhängen. Die täglich zu verabreichende Dosis wirksamer Substanz liegt bei 0.01 mg bis ungefähr 100 mg/kg Körpergewicht, vorzugsweise bei 0.1 bis 10 mg/kg Körpergewicht und kann auf einmal oder mehrere Male verteilt appliziert werden.

Besonders bevorzugt sind außer den in den Beispielen aufgeführten Verbindungen die folgenden Verbindungen:

1) 2-Amino-6-methylcarbamoylmethyl-azulen-1,3-dicarbonsäuredimethylester
2) 2-Acetylamino-6-methoxycarbonylmethyl-azulen-1,3-dicarbonsäuredimethylester
3) 2-Acetylamino-6-hydroxycarbamoylmethyl-azulen-1,3-dicarbonsäuredimethylester
4) 2-Acetylamino-6-mercaptomethyl-azulen-1,3-dicarbonsäuredimethylester
5) 2-Acetylamino-6-phosphonomethyl-azulen-1,3-dicarbonsäuredimethylester
6) 2-Acetylamino-6-carboxymethyl-azulen-1,3-dicarbonsäuredimethylester
7) 2-Acetylamino-6-(hydroxy-isopropyl-phosphinoylmethyl)-azulen-1,3-dicarbonsäuredimethylester
8) 2-Acetylamino-6-[(formyl-hydroxy-amino)-methyl]-azulen-1,3-dicarbonsäuredimethylester
9) 2-Acetylamino-6-(2,4,6-trioxo-hexahydro-pyrimidin-5-ylmethyl)-azulen-1,3-dicarbonsäuredimethylester
10) 2-(1,3-Dioxo-1,3-dihydro-isoindol-2-yl)-6-methoxycarbonylmethyl-azulen-1,3-dicarbonsäurediethylester
11) 2-(1,3-Dioxo-1,3-dihydro-isoindol-2-yl)-6-hydroxycarbamoylmethyl-azulen-1,3-dicarbonsäurediethylester
12) 2-(1,3-Dioxo-1,3-dihydro-isoindol-2-yl)-6-mercaptomethyl-azulen-1,3-dicarbonsäurediethylester
13) 2-(1,3-Dioxo-1,3-dihydro-isoindol-2-yl)-6-phosphonomethyl-azulen-1,3-dicarbonsäurediethylester
14) 6-Carboxymethyl-2-(1,3-dioxo-1,3-dihydro-isoindol-2-yl)-azulen-1,3-dicarbonsäurediethylester
15) 2-(1,3-Dioxo-1,3-dihydro-isoindol-2-yl)-6-(hydroxy-isopropyl-phosphinoylmethyl)azulen-1,3-dicarbonsäurediethylester
16) 2-(1,3-Dioxo-1,3-dihydro-isoindol-2-yl)-6-[(formyl-hydroxy-amino)-methyl]-azulen-1,3-dicarbonsäurediethylester
17) 2-(1,3-Dioxo-1,3-dihydro-isoindol-2-yl)-6-(2,4,6-trioxo-hexahydro-pyrimidin-5-yl-methyl)-azulen-1,3-dicarbonsäurediethylester
18) 2-(1,3-Dioxo-1,3-dihydro-isoindol-2-yl)-6-methoxycarbonylmethyl-3-propyl-azulen-1-carbonsäureethylester
19) 2-(1,3-Dioxo-1,3-dihydro-isoindol-2-yl)-6-hydroxycarbamoylmethyl-3-propylazulen-1-carbonsäureethylester
20) 2-(1,3-Dioxo-1,3-dihydro-isoindol-2-yl)-6-mercaptomethyl-3-propyl-azulen-1-carbonsäureethylester
21) 3-Butyl-2-(1,3-dioxo-1,3-dihydro-isoindol-2-yl)-6-methoxycarbonylmethyl-azulen-1-carbonsäureethylester
22) 3-Butyl-2-(1,3-dioxo-1,3-dihydro-isoindol-2-yl)-6-hydroxycarbamoylmethyl-azulen-1-carbonsäureethylester
23) 3-Butyl-2-(1,3-dioxo-1,3-dihydro-isoindol-2-yl)-6-mercaptomethyl-azulen-1-carbonsäureethylester
24) 2-(1,3-Dioxo-1,3-dihydro-isoindol-2-yl)-6-methoxycarbonylmethyl-3-pentyl-azulen-1-carbonsäureethylester
25) 2-(1,3-Dioxo-1,3-dihydro-isoindol-2-yl)-6-hydroxycarbamoylmethyl-3-pentyl-azulen-1-carbonsäureethylester
26) 2-(1,3-Dioxo-1,3-dihydro-isoindol-2-yl)-6-mercaptomethyl-3-pentyl-azulen-1-carbonsäureethylester
27) 2-(1,3-Dioxo-1,3-dihydro-isoindol-2-yl)-3-hexyl-6-methoxycarbonylmethyl-azulen-1-carbonsäureethylester
28) 2-(1,3-Dioxo-1,3-dihydro-isoindol-2-yl)-3-hexyl-6-hydroxycarbamoylmethyl-azulen-1-carbonsäureethylester
29) 2-(1,3-Dioxo-1,3-dihydro-isoindol-2-yl)-3-hexyl-6-mercaptomethyl-azulen-1-carbonsäureethylester
30) 2-(1,3-Dioxo-1,3-dihydro-isoindol-2-yl)-3-isobutyl-6-methoxycarbonylmethyl-azulen-1-carbonsäureethylester
31) 2-(1,3-Dioxo-1,3-dihydro-isoindol-2-yl)-6-hydroxycarbamoylmethyl-3-isobutyl-azulen-1-carbonsäureethylester
32) 2-(1,3-Dioxo-1,3-dihydro-isoindol-2-yl)-3-isobutyl-6-mercaptomethyl-azulen-1-carbonsäureethylester
33) 2-(1,3-Dioxo-1,3-dihydro-isoindol-2-yl)-6-methoxycarbonylmethyl-3-(3-methylbutyl)-azulen-1-carbonsäureethylester
34) 2-(1,3-Dioxo-1,3-dihydro-isoindol-2-yl)-6-hydroxycarbamoylmethyl-3-(3-methyl-butyl)-azulen-1-carbonsäureethylester
35) 2-(1,3-Dioxo-1,3-dihydro-isoindol-2-yl)-6-mercaptomethyl-3-(3-methyl-butyl)-azulen-1-carbonsäureethylester
36) 2-Acetylamino-6-methoxycarbonylmethy-3-propyl-azulen-1-carbonsäureethylester
37) 2-Acetylamino-6-hydroxycarbamoylmethyl-3-propyl-azulen-1-carbonsäureethylester
38) 2-Acetylamino-6-mercaptomethyl-3-propyl-azulen-1-carbonsäureethylester

39) 2-Acetylamino-3-butyl-6-methoxycarbonylmethyl-azulen-1-carbonsäureethylester
40) 2-Acetylamino-3-butyl-6-hydroxycarbamoylmethyl-azulen-1-carbonsäureethylester
41) 2-Acetylamino-3-butyl-6-mercaptomethyl-azulen-1-carbonsäureethylester
42) 2-Acetylamino-6-methoxycarbonylmethyl-3-pentyl-azulen-1-carbonsäureethylester
43) 2-Acetylamino-6-hydroxycarbamoylmethyl-3-pentyl-azulen-1-carbonsäureethylester
44) 2-Acetylamino-6-mercaptomethyl-3-pentyl-azulen-1-carbonsäureethylester
45) 2-Acetylamino-3-hexyl-6-methoxycarbonylmethyl-azulen-1-carbonsäureethylester
46) 2-Acetylamino-3-hexyl-6-hydroxycarbamoylmethyl-azulen-1-carbonsäureethylester
47) 2-Acetylamino-3-hexyl-6-mercaptomethyl-azulen-1-carbonsäureethylester
48) 2-Acetylamino-3-isobutyl-6-methoxycarbonylmethyl-azulen-1-carbonsäureethylester
49) 2-Acetylamino-6-hydroxycarbamoylmethyl-3-isobutyl-azulen-1-carbonsäureethylester
50) 2-Acerylamino-3-isobutyl-6-mercaptomethyl-azulen-1-carbonsäureethylester
51) 2-Acerylamino-6-methoxycarbonylmethyl-3-(3-methyl-butyl)-azulen-1-carbonsäureethylester
52) 2-Acetylamino-6-hydroxycarbamoylmethyl-3-(3-methyl-butyl)-azulen-1-carbonsäureethylester
53) 2-Acetylamino-6-mercaptomethyl-3-(3-methyl-butyl)-azulen-1-carbonsäureethylester
54) 6-Methoxycarbonylmethyl-3-propyl-azulen-1-carbonsäureethylester
55) 6-Hydroxycarbamoylmethyl-3-propyl-azulen-1-carbonsäureethylester
56) 6-Mercaptomethyl-3-propyl-azulen-1-carbonsäureethylester
57) 3-Butyl-6-methoxycarbonylmethyl-azulen-1-carbonsäureethylester
58) 3-Butyl-6-hydroxycarbamoylmethyl-azulen-1-carbonsäureethylester
59) 3-Butyl-6-mercaptomethyl-azulen-1-carbonsäureethylester
60) 6-Hydroxycarbamoylmethyl-3-pentyl-azulen-1-carbonsäureethylester
61) 6-Mercaptomethyl-3-pentyl-azulen-1-carbonsäureethylester
62) 3-Hexyl-6-methoxycarbonylmethyl-azulen-1-carbonsäureethylester
63) 3-Hexyl-6-hydroxycarbamoylmethyl-azulen-1-carbonsäureethylester
64) 3-Hexyl-6-mercaptomethyl-azulen-1-carbonsäureethylester
65) 3-Isobutyl-6-methoxycarbonylmethyl-azulen-1-carbonsäureethylester
66) 6-Hydroxycarbamoylmethyl-3-isobutyl-azulen-1-carbonsäureethylester
67) 3-Isobutyl-6-mercaptomethyl-azulen-1-carbonsäureethylester
68) 6-Methoxycarbonylmethyl-3-(3-methyl-butyl)-azulen-1-carbonsäureethylester
69) 6-Hydroxycarbamoylmethyl-3-(3-methyl-butyl)-azulen-1-carbonsäureethylester
70) 6-Mercaptomethyl-3-(3-methyl-butyl)-azulen-1-carbonsäureethylester
71) 6-Methoxycarbonylmethyl-3-propoxy-azulen-1-carbonsäureethylester
72) 6-Hydroxycarbamoylmethyl-3-propoxy-azulen-1-carbonsäureethylester
73) 6-Mercaptomethyl-3-propoxy-azulen-1-carbonsäureethylester
74) 2-(4-Butoxy-6-hydroxycarbamoylmethyl-azulen-1-yl)-acetamide
75) 3-(4-Butoxy-1-carbamoylmethyl-azulen-6-yl)-*N*-hydroxy-*N*-methyl-propionamide
76) (4-Butoxy-1-carbamoylmethyl-azulen-6-ylmethyl)-phosphonsäurediethylester
77) 2-(4-Butoxy-azulen-6-yl)-*N*-hydroxy-acetamide
78) (4-Butoxy-azulen-6-yl)-essigsäure
79) 3-(4-Butoxy-azulen-6-yl)-*N*-hydroxy-*N*-methyl-propionamide
80) (4-Propoxy-azulen-6-yl)-essigsäure
81) (4-Pentyloxy-azulen-6-yl)-essigsäure
82) (4-Butoxy-1-carbamoylmethyl-azulen-6-ylmethyl)-phosphonsäure
83) (4-Butoxy-1-carbamoylmethyl-azulen-6-ylmethyl)-phosphonsäurediethylester
84) (4-Butoxy-azulen-6-ylmethyl)-phosphonsäure
85) (4-Butoxy-azulen-6-ylmethyl)-phosphonsäurediethylester
86) 2-(1,3-Dioxo-1,3-dihydro-isoindol-2-yl)-azulen-1,3,5-tricarbonsäure 1,3-diethylester-5-methylester
87) 2-(1,3-Dioxo-1,3-dihydro-isoindol-2-yl)-5-hydroxycarbamoyl-azulen-1,3-dicarbonsäurediethylester
88) 2-(1,3-Dioxo-1,3-dihydro-isoindol-2-yl)-5-mercapto-azulen-1,3-dicarbonsäurediethylester
89) 2-(1,3-Dioxo-1,3-dihydro-isoindol-2-yl)-5-phosphono-azulen-1,3-dicarbonsäurediethylester
90) 2-(1,3-Dioxo-1,3-dihydro-isoindol-2-yl)-azulen-1,3,5-tricarbonsäure-1,3-diethylester
91) 2-(1,3-Dioxo-1,3-dihydro-isoindol-2-yl)-5-(hydroxy-isopropyl-phosphinoyl)-azulen-1,3-dicarbonsäurediethylester
92) 2-(1,3-Dioxo-1,3-dihydro-isoindol-2-yl)-5-(formyl-hydroxy-amino)-azulen-1,3-dicarbonsäurediethylester
93) 2-(1,3-Dioxo-1,3-dihydro-isoindol-2-yl)-5-(2,4,6-trioxo-hexahydro-pyrimidin-5-yl)-azulen-1,3-dicarbonsäurediethylester
94) 2-(1,3-Dioxo-1,3-dihydro-isoindol-2-yl)-5-[(hydroxy-methyl-carbamoyl)-methyl]-azulen-1,3-dicarbonsäurediethylester
95) 5-(Diethoxy-phosphoryl)-2-(1,3-dioxo-1,3-dihydro-isoindol-2-yl)-azulen-1,3-dicarbonsäurediethylester
96) 5-(Diethoxy-phosphoryl)-2-(1,3-dioxo-1,3-dihydro-isoindol-2-yl)-azulen-1,3-dicarbonsäurediethylester
97) 5-Hydroxycarbamoyl-3-propyl-azulen-1-carbonsäureethylester
98) 5-Mercapto-3-propyl-azulen-1-carbonsäureethylester
100) 3-Butyl-azulen-1,5-dicarbonsäure1-ethylester 5-methylester
101) 3-Butyl-5-hydroxycarbamoyl-azulen-1-carbonsäureethylester
102) 3-Butyl-5-mercapto-azulen-1-carbonsäureethylester
103) 3-Pentyl-azulen-1,5-dicarbonsäure1-ethylester 5-methylester
104) 5-Hydroxycarbamoyl-3-pentyl-azulen-1-carbonsäureethylester
105) 5-Mercapto-3-pentyl-azulen-1-carbonsäureethylester
106) 3-Hexyl-azulen-1,5-dicarbonsäure1-ethylester 5-methylester
107) 3-Hexyl-5-hydroxycarbamoyl-azulen-1-carbonsäureethylester
108) 3-Hexyl-5-mercapto-azulen-1-carbonsäureethylester
109) 3-Isobutyl-azulen-1,5-dicarbonsäure1-ethylester 5-methylester
110) 5-Hydroxycarbamoyl-3-isobutyl-azulen-1-carbonsäureethylester
111) 3-Isobutyl-5-mercapto-azulen-1-carbonsäureethylester
112) 3-(3-Methyl-butyl)-azulen-1,5-dicarbonsäure1-ethylester 5-methylester
113) 5-Hydroxycarbamoyl-3-(3-methyl-butyl)-azulen-1-carbonsäureethylester
114) 5-Mercapto-3-(3-methyl-butyl)-azulen-1-carbonsäureethylester
115) 3-Propyl-azulen-5-carbonsäuremethylester
116) 3-Propyl-azulen-5-carbonsäurehydroxyamide
117) 3-Propyl-azulen-5-thiol
118) 3-Butyl-azulen-5-carbonsäuremethylester
119) 3-Butyl-azulen-5-carbonsäurehydroxyamide
120) 3-Butyl-azulen-5-thiol
121) 3-Pentyl-azulen-5-carbonsäuremethylester
122) 3-Pentyl-azulen-5-carbonsäurehydroxyamide
123) 3-Pentyl-azulen-5-thiol
124) 3-Hexyl-azulen-5-carbonsäuremethylester
125) 3-Hexyl-azulen-5-carbonsäurehydroxyamide
126) 3-Hexyl-azulen-5-thiol
127) 3-Isobutyl-azulen-5-carbonsäuremethylester
128) 3-Isobutyl-azulen-5-carbonsäurehydroxyamide
129) 3-Isobutyl-azulen-5-thiol
130) 3-(3-Methyl-butyl)-azulen-5-carbonsäuremethylester
131) 3-(3-Methyl-butyl)-azulen-5-carbonsäurehydroxyamide
132) 3-(3-Methyl-butyl)-azulen-5-thiol

Verbindungen der allgemeinen Formel I bzw. II in denen die Reste $R_1$ bis $R_6$ die angegebene Bedeutung haben können nach an sich literaturbekannten Verfahren dargestellt werden (siehe K.-P. Zeller „Azulene" in Methoden der organischen Chemie (Houben - Weyl), Band V/2c, Seite 127).
Z. B. werden solche Verbindungen aus entsprechend substituierten Troponen (Siehe zur Synthese: T. Asao, M. Oda, „Tropone, Tropolone und deren Derivate" in Methoden der organischen Chemie (Houben -Weyl), Band V/2c Seite 710) erhalten. (siehe z. B.: T. Nozoe, H. Takeshita, K. Tajiri; *Bull. Chem. Soc. Jpn.* **56**, 3679 und darin zitierte Literatur)

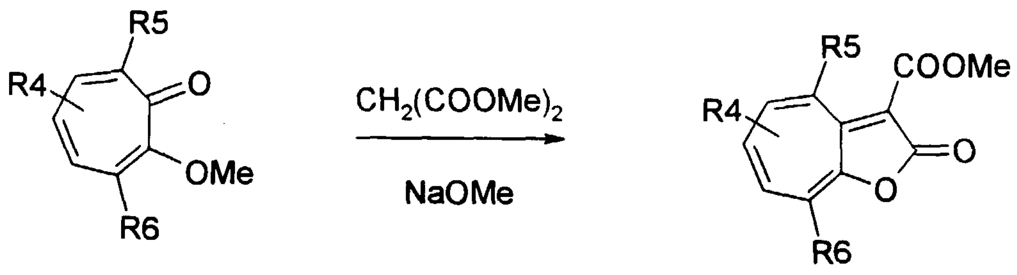

Die so erhaltenen 2H-Cyclohepta [b] furan-2-one lassen sich mit aktiven Methylencomponenten (z. B. Essigsäurederivaten (T. Nozoe, K. Takase, T. Nakazawa, S. Sugita, *Bull. Chem. Soc Jap.* **1974**, 1750 und dort zitierte Literatur) oder Enaminen (z. B. M. Yasunami, A. Chen, P. W. Wang, K. Takase, *Chem. Lett.* **1980**, 579)) zu den entsprechenden Azulenen umsetzen.

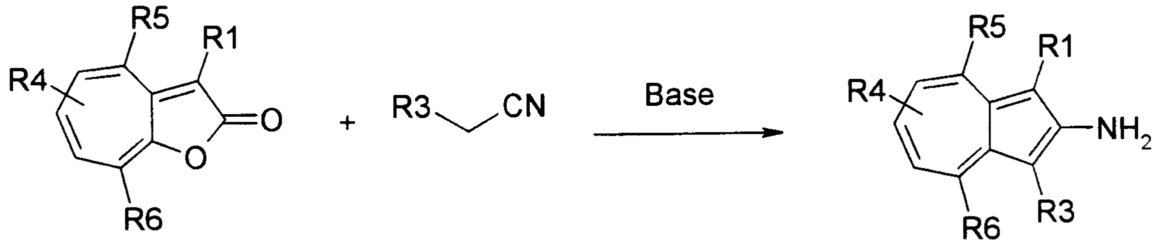


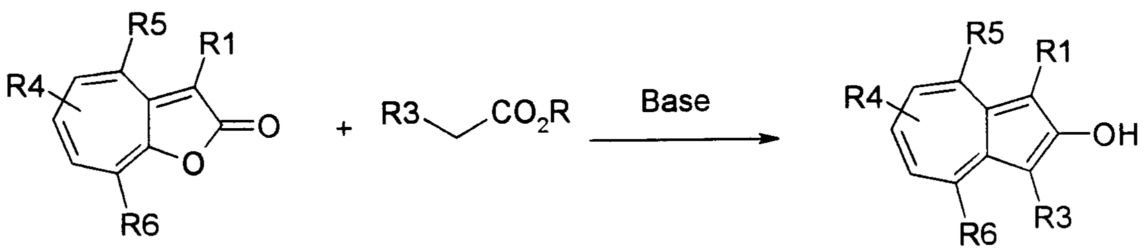


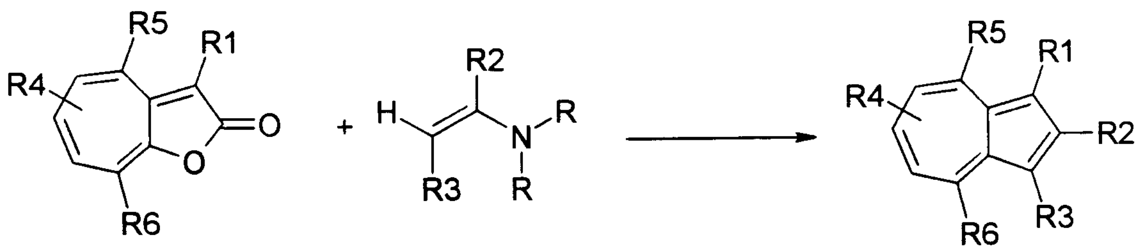

Die so erhaltenen Aminoazulene können nach an sich bekannten Verfahren zu verschiedenen Azulenderivaten in denen die Reste $R_1$-$R_6$ die angegebene Bedeutung haben umgesetzt werden. Beispielsweise kann nach Nitrosierung die Aminogruppe durch Halogen ersetzt werden (z. B.: T. Nozoe, S. Seto, S. Matsumura, *Bull. Chem. Soc. Jap.* **1962**, 1990). Auch die gezeigten Hydroxyderivate in denen Die Reste $R_1$ und $R_3$-$R_6$ die angegebene Bedeutung haben, sind vielseitige Ausgangsstoffe. Sie reagieren nach Methylierung z. B. mit Grignard-Verbindungen zu den entsprechenden alkylierten Produkten (D. Balschukat, E. V. Dehmlow, *Chem. Ber.* **1986**, 2272).

Die folgenden Beispiele legen die Erfindung exemplarisch dar:

**Beispiel 1:**

2-Amino-6-carboxylmethyl-azulen-1,3-dicarbonsäurediethylester

Die Synthese von 2-Amino-6-ethoxycarbonylmethyl-azulen-1,3-dicarbonsäurediethylester ist literaturbekannt (Dehmlow, Eckehard V.; Balschukat, Dietmar; Schmidt, Peter P.; Groening, Carsten; *Chem.Ber.;* **1986**; 2956). 0.1g 2-Amino-6-ethoxycarbonylmethyl-azulen-1,3-dicarbonsäurediethylester werden in 3 ml Ethanol gelöst und 0.29 ml 2N NaOH zugegeben. Es wird 30 min. bei 60 °C gehalten. Nach Verdünnen mit Wasser wird mit 2N Schwefelsäure angesäuert und mit Essigester extrahiert. Die vereinigten organischen Phasen werden über Natriumsulfat getrocknet und am Vakuum vom Lösungsmittel befreit. Man erhält 85 mg (91.9%) der Carbonsäure.

Schmp.: 215-217 °C

**Beispiel 2**

2-Amino-6-carboxylmethyl-azulen-1,3-dicarbonsäuremonoethylester

85 mg 2-Amino-6-carboxylmethyl-azulen-1,3-dicarbonsäurediethylester werden 3 ml Ethanol und 3 ml Wasser gelöst und 0.369 ml 2 N Natronlauge zugegeben. Es wird 8 h am Rückfluß gehalten und der Ansatz anschließend auf verdünnte Schwefelsäure gegossen. Es wird mit Essigester extrahiert und die vereinigten organischen Phasen am Vakuum vom Lösungsmittel befreit. Man erhält 34 mg (43%) der Dicarbonsäuremonoethylester.

Schmp.: 149-150 °C

**Beispiel 3**

2-Amino-6-hydroxycarbamoylmethyl-azulen-1,3-dicarbonsäurediethylester

150 mg 2-Amino-6-carboxylmethyl-azulen-1,3-dicarbonsäurediethylester werden in 2 ml THF gelöst und auf 45 °C erwärmt. Dann werden 70.4 mg Carbonyldiimidazol portionsweise zugegeben. Es wird 30 min. bei 45 °C gerührt, wobei sich ein Niederschlag bildet. Es wird mit 2 ml THF verdünnt, 31 mg Hydroxylaminhydrochlorid zugegeben und 16 h am Rückfluß gehalten. Das ausgefallene Produkt wird mit Ethanol versetzt erwärmt und heiß abgesaugt. Man erhält 48 mg (31%) 2-Amino-6-hydroxy-carbamoylmethyl-azulen-1,3-dicarbonsäurediethylester.

Schmp.: 195-197 °C

**Beispiel 4**

2-Acetylamino-6-carboxymethyl-azulen-1,3-dicarbonsäurediethylester

1) 2-Diacetylamino-6-ethoxycarbonylmethyl-azulen-1,3-dicarbonsäurediethylester

1.5 g 2-Amino-6-ethoxycarbonylmethyl-azulen-1,3-dicarbonsäurediethylester werden in 5 ml Acetanhydrid gelöst und 3h bei 145°C gerührt und eirrotiert. Das Acetanhydrid wird abdestilliert und der Rückstand aus Ethanol umkristallisiert. Man erhält 1.5 g 2-Diacetylamino-6-ethoxycarbonylmethyl-azulen-1,3-dicarbonsäurediethylester (82%). Schmp.: 123-125 °C

2) 2-Acetylamino-6-carboxymethyl-azulen-1,3-dicarbonsäurediethylester

300 mg 2-Diacetylamino-6-ethoxycarbonylmethyl-azulen-1,3-dicarbonsäurediethylester werden in 9 ml Ethanol gelöst und 0.66 ml 2N Natromlauge zugegeben. Es wird 40 min. bei 60 °C gerührt und einrotiert. Das Rohprodukt wird mit Essigester gewaschen und in Wasser aufgenommen und mit verdünnter Salzsäure angesäuert. Das ausgefallene Produkt wird in Essigester gelöst, über Natriumsulfat getrocknet und i.V. vom Lösungsmittel befreit. Man erhält 153 mg der Monoacetylverbindung (60%).

Schmp.: 218-221 °C

**Beispiel 5**

2-Acetylamino-6-hydroxycarbamoylmethyl-azulen-1,3-dicarbonsäurediethylester

130 mg 2-Acetylamino-6-ethoxycarbonylmethyl-azulen-1,3-dicarbonsäurediethylester werden in 5 ml THF gelöst und auf 45 °C erwärmt. Dann werden 60 mg Carbonyldiimidazol portionsweise zugegeben und 30 min. bei 45 °C gerührt. Es wird eine Spatelspitze Dimethylaminopyridin und 36 mg Hydroxylaminhydrochlorid zugegeben und 16 h bei 70 °C gerührt. Nach Abkühlen wird das ausgefallene Produkt abgesaugt und mit Ether gewaschen. Das Produkt wird mittels Reversed Phase Chromatographle an einer RP 18 Säule gereinigt (Laufmittel: Methanol/Wasser 70/30). Man erhält12.8 mg (10%) der gewünschten Verbindung.

Schmp.: 197-199 °C

**Beispiel 6**

2-Amino-6-(1-ethoxycarbonyl-2-oxo-2-ureido-ethyl)-azulen-1,3-dicarbonsäurediethylester

Die Synthese von 6-(Bis-ethoxycarbonyl-methyl)-2-diacetylamino-azulene-1,3-dicarbonsäurediethylester ist literaturbekannt (Dehmlow, Eckehard V.; Balschukat, Dietmar; Schmidt, Peter P.; Groening, Carsten; *Chem.Ber.* 119, **1986**, 2956-2962). 233 mg dieser Verbindung und 40 mg Harnstoff werden zu einer Lösung aus 20 mg Natrium in 3 ml Ethanol gegeben und 4h am Rückfluß gehalten. Das ausgefallene Produkt wird abgesaugt und mit Ethanol gewaschen. Es wurde in warmem Wasser gelöst und mit 6 N Salzsäure auf pH 1 gebracht. Die ausgefallene Substanz wurde abgesaugt und mit Ethanol und Isohexan gewaschen. Man erhält 53 mg (26%) 2-Amino-6-(1-ethoxycarbonyl-2-oxo-2-ureido-ethyl)-azulen-1,3-dicarbonsäurediethylester.

Schmp.: 226-228 °C

**Beispiel 7**

2-Amino-azulen-1,3-dicarbonsäure-di-*tert*-butylester

900 mg Kalium-*tert*-butylat werden bei 30 °C in 10 ml *tert*-Butanol gelöst, nach 5 min. 1.1g *tert*-Butylcyanoacetat zugegeben und weitere 30 min bei 30 °C gerührt. Dann werden 500 mg 2-Chlortropolon zugegeben und 3 h gerührt. Die Lösung wird eingeengt, mit Ether verdünnt, mit Wasser gewaschen, die vereinigten organischen Phasen über Natriumsulfat getrocknet und am Vakuum vom Lösungsmittel befreit. Man erhält ein braunschwarzes Öl, das vermittels Flashchromatographie (Heptan/Essigester 8/1) gereinigt wird. Dies ergibt 800 mg 2-Amino-azulen-1,3-dicarbonsäure-di-*tert*-butylester (67%).

Schmp.: 104-107 °C

**Beispiel 8**

Bei Durchführung obiger Reaktion in Ethanol wird neben dem erwünschten Produkt auch 2-Amino-azulen-1,3-dicarbonsäure-*tert*-butyl-ethylester erhalten.

Schmp.: 90-92 C

**Beispiel 9**

2-Amino-6-bromo-azulen-1,3-dicarbonsäure-di-*tert*-butylester

500 mg 2-Amino-azulen-1,3-dicarbonsäure-di-*tert*-butylester werden in 10 ml Chloroform gelöst, mit 200 mg Brom versetzt und 123 mg Natriumhydrogencarbonat zugegeben. Die Reaktion wird 3d bei RT gerührt. Die Lösung wird eingeengt, mit Methylenchlorid verdünnt und mit Wasser gewaschen. Die vereinigten organischen Phasen werden über Natriumsulfat getrocknet und i.V. vom Lösungsmittel befreit. Das Produkt wird durch Chromatograhie an Kieselgel (Toluol) gereinigt. Man erhält 280 mg (46%) des gewünschten Produktes.

Schmp.: 193 °C

**Beispiel 10**

2-Diacetylamino-6-bromo-azulen-1,3-dicarbonsäure-di-*tert*-butylester

250 mg 2-Amino-6-bromo-azulen-1,3-dicarbonsäure-di-*tert*-butylester werden in 1 ml Acetanhydrid gelöst und 15 h am Rückfluß gehalten. Das Acetanhydrid wird am Vakuum entfernt und das Produkt mit Heptan gewaschen. Man erhält 185 mg (62%) 2-Diacetylamino-6-bromo-azulen-1,3-dicarbonsäure-di-*tert*-butylester

Schmp.: 175-178 °C

**Beispiel 11**

6-(Bis-*tert*-butoxycarbonyl-methyl)-2-diacetylamino-azulene-1,3-dicarbonsäure-di-*tert*-butylester

Zu einer Lösung von 362 mg Kalium-*tert*-butylat in 10 ml *tert*-Butanol wird bei 35 °C 1.4 ml Malonsäuredi-*tert*-butylester zugetropft, wobei ein weißer Niederschlag ausfällt. Nach 20 min. Rühren bei 35 °C wird eine Lösung von 500 mg 2-Diacetylamino-6-bromo-azulen-1,3-dicarbonsäure-di-*tert*-butylester in 8 ml *tert*-Butanol zugetropft und 20 h bei RT gerührt. Die Lösung wird mit Wasser verdünnt und mit 2N Schwefelsäure auf pH 1 gebracht. Es wird mit Methylenchlorid extrahiert, die vereinigten organischen Phasen über Natriumsulfat getrocknet und i. V. vom Lösungsmittel befreit. Der ölige Rückstand wird durch Chromatographie an Kieselgel (Heptan/Essigester 3:1) gereinigt. Man erhält das gewünschte Produkt und die monoacetylierte Verbindung im Verhältnis 1:1 . 490 mg (78%)

Schmp.: 90-92 °C

**Beispiel 12**

Periphere humane Leukozyten werden mit LPS über 22 Std. inkubiert. Der Kulturüberstand wird mit einem TNF$\alpha$ spezifischen Enzym-Immuno-Assay untersucht.

Durch Vergleich der für die Messproben erhaltenen TNF$\alpha$-Konzentrationen mit den ungehemmten Proben wird die prozentuale Hemmung fiir die einzelne Probe berechnet. Aus der Konzentrationsabhängigkeit der Hernmwerte werden rechnerisch die $IC_{50}$-Werte bestimmt.

Der $IC_{50}$-Wert läßt sich wie folgt ermitteln:

$$v = v_0/(1 + [I] / IC_{50})$$

v = Anfangsgechwindigkeit
$V_0$ = Anfangsgeschwindigkeit ohne Inhibitor
[I] = Inhibitorkonzentration

Die $IC_{50}$-Werte, der in den Beispielen beschriebenen Substanzen gegen TNF-$\alpha$ finden sich in der folgenden Tabelle:

| Beispiel | $IC_{50}$ [$\mu$M] |
|---|---|
| 1 | 0.16 |
| 2 | 10.5 |
| 3 | 0.001 |
| 4 | 4.1 |
| 5 | 0.02 |
| 6 | 15.0 |
| 7 | 2.8 |
| 8 | 8.0 |
| 9 | 2.3 |
| 10 | 0.5 |
| 11 | 4.2 |

**Patentansprüche**

**1.** Verbindungen der Formel I bzw. II

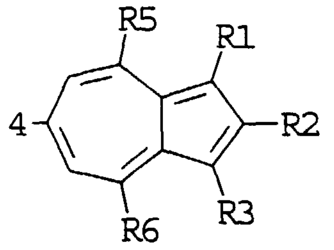

I

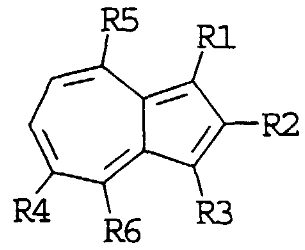

II

in der

$R_1$, $R_2$ und $R_3$ einzeln oder unabhängig voneinander
Wasserstoff, Hydroxy-, Amino-, Mercapto-, ein gerader oder verzweigter, gesättigter oder ungesättigter aliphatischer Rest mit 1-15 Kohlenstoffatomen, der ein oder mehrfach substituiert sein kann, eine Alkoxy-, Alkylamino-, Di- Alkylamino-, Alkylmercapto-, Alkylsulfinyl-, Alkylsufonyl-, Alkenyl-, Alkinyl-, Alkenyloxy-, Alkenylmercapto-, Alkinyloxy-, Alkinylmercapto-, Alkylcarbonylamino-, Di-N-Alkylcarbonylamino-, Alkylaminocarbonyl-, Formyl-, Alkylcarbonyl-, Carboxyl-, Alkoxycarbonyl-, Alkenyloxycarbonyl-, Alkinyloxycarbonyl-, Carboxyalkyl-, Alkyloxycarbonylalkyl, Alkenyloxycarbonylalkyl-, Alkinyloxycarbonylalkyl-, Nitro-, Cyano-, Halogen-, Trifluormethyl-, Azidogruppe, Phenyl, oder Phenylcarbonyl, das gegebenenfalls ein oder mehrfach unabhängig voneinander substituiert sein kann, einen carbocyclischen mono-, bi- oder tricyclischen Rest mit 7-15 C-Atomen oder ein heterocyclisches mono-, bi- oder tricyclisches Ringsystem bedeuten,

$R_4$ im Fall von Verbindungen der allgemeinen Formel I
$(CH_2)_nCH_2R_7$, $(CH_2)_nCHR_7R_8$, $CH(OR_8)R_7$, $CH(SR_8R_7$, $NR_9R_{10}$, $OR_{10}$, $SR_{10}$, $C(O)R_{10}$,

im Fall von Verbindungen der allgemeinen Formel II
$R_7$, $(CH_2)_nCH_2R_7$, $(CH_2)_nCHR_7R_8$, $CH(OR_8)R_7$, $CH(SR_8)R_7$, $NR_9R_{10}$, $OR_{10}$, $SR_{10}$, $C(O)R_{10}$,

n= die Zahl 0 oder 1

$R_5$ und $R_6$ einzeln und unabhängig voneinander
Wasserstoff, ein gerader oder verzweigter, gesättigter oder ungesättigter aliphatischer Rest mit 1-15 Kohlenstoffatomen, der ein oder mehrfach substituiert sein kann, eine Alkoxy-, Alkylamino-, Di- Alkylamino-, Alkylmercapto-, Alkylsulfinyl-, Alkylsufonyl-, Alkenyl-, Alkinyl-, Alkenyloxy-, Alkenylmercapto-, Alkinyloxy-, Alkinylmercapto-, Alkylcarbonylamino-, Di- -Alkylcarbonylamino-, Alkylaminocarbonyl-, Formyl-, Alkylcarbonyl-, Carboxyl-, Alkoxycarbonyl-, Alkenyloxycarbonyl-, Alkinyloxycarbonyl-, Carboxyalkyl-, Alkyloxycarbonylalkyl, Alkenyloxycarbonylalkyl-, Alkinyloxycarbonylalkyl-, Nitro-, Cyano-, Halogen-, Trifluormethyl-, Azidogruppe, Phenyl, oder Phenylcarbonyl, das gegebenenfalls ein oder mehrfach unabhängig voneinander substituiert sein kann, einen carbocyclischen mono-, bi- oder tricyclischen Rest mit 7-15 C-Atomen oder ein heterocyclisches mono-, bi- oder tricyclisches Ringsystem,

$R_7$,
Carboxyl-, Carboxoureido-, Alkoxycarbonyl-, Alkenyloxycarbonyl-, Alkinyloxycarbonyl-, Aminocarbonyl-, Alkylaminocarbonyl-, Alkenyloxycarbonylalkyl-, Alkinyloxycarbonylalkyl-, *N*-Hydroxyaminocarbonyl-, *N*-Alkoxyaminocarbonyl-, *N*-Alkyl-*N*-Hydroxyaminocarbonyl-, *N*-Alkyl-*N*-Alkoxyaminocarbonyl-, Mercapto-, Alkylmercapto-, Phenylmercapto-, Pyridylmercapto, Halogen-, Dihydroxyboryl-, Di-Alkoxyboryl-, Sulfonsäure-, Phosphonsäure-, Barbitursäure-,

$R_8$
Wasserstoff, Alkyl-, Alkenyl-, Alkinyl-, Phenyl-, Pyridyl-, Thiophenyl-, Alkoxycarbonyl-,
und

$R_9$
Wasserstoff, Benzyl, Phenyl, Alkyl bedeutet,
und

$R_{10}$
Wasserstoff, Carboxyl-, Alkoxycarbonyl-, Alkenyloxycarbonyl-, Alkinyloxycarbonyl-, Aminocarbonyl-, Alkylaminocarbonyl-, Alkenyloxycarbonylalkyl-, Alkinyloxycarbonylalkyl-, *N*-Hydroxyaminocarbonyl-, *N*-Alkoxyaminocarbonyl-, *N*-Alkyl-*N*-Hydroxyaminocarbonyl-, N-Alkyl-*N*-Alkoxyaminocarbonyl- bedeutet
wobei mindestens einer der Reste $R_1$, $R_3$, $R_5$ oder $R_6$ ungleich Wasserstoff sein muß,

sowie deren Tautomere, Enantiomere, Diastereomere, Racemate und physiologisch verträglichen Salze oder Ester und Substanzen, die *in vivo* zu Verbindungen der Formel I bzw. II hydrolysiert oder metabolisiert werden.

**2.** Verbindungen, ausgewählt aus der Gruppe

2-Amino-6-carboxylmethyl-azulen-1,3-dicarbonsäurediethylester
2-Amino-6-carboxylmethyl-azulen-1,3-dicarbonsäuremonoethylester
2-Amino-6-hydroxycarbamoylmethyl-azulen-1,3-dicarbonsäurediethylester
2-Acetylamino-6-carboxymethyl-azulen-1,3-dicarbonsäurediethylester
2-Acetylamino-6-hydroxycarbamoylmethyl-azulen-1,3-dicarbonsäurediethylester
2-Amino-6-(1-ethoxycarbonyl-2-oxo-2-ureido-ethyl)-azulen-1,3-dicarbonsäurediethylester
2-Amino-azulen-1,3-dicarbonsäure-di-*tert*-butylester
2-Amino-azulen-1,3-dicarbonsäure-*tert*-butyl-ethylester
2-Amino-6-bromo-azulen-1,3-dicarbonsäure-di-*tert*-butylester
2-Diacetylamino-6-bromo-azulen-1,3-dicarbonsäure-di-*tert*-butylester
6-(Bis-*tert*-butoxycarbonyl-methyl)-2-diacetylamino-azulene-1,3-dicarbonsäure-di-*tert*-butylester

sowie deren Tautomere, Enantiomere, Diastereomere, Racemate und physiologisch verträglichen Salze oder Ester.

**3.** Arzneimittel, enthaltend mindestens eine Verbindung der allgemeinen Formel I bzw. II, gemäß Anspruch 1 oder 2 neben üblichen pharmazeutischen Hilfs- und Trägerstoffen.

**4.** Verwendung von Verbindungen gemäß Anspruch 3 oder 4 zur Herstellung von Arzneimittelformulierungen mit Metalloproteinase-inhibierender Wirkung.

**5.** Pharmazeutische Zubereitung, dadurch gekennzeichnet, daß sie ein Arzneimittel gemäß Ansptuch 3 enthält.

Europäisches Patentamt

# EUROPÄISCHER TEILRECHERCHENBERICHT

der nach Regel 45 des Europäischen Patentübereinkommens für das weitere Verfahren als europäischer Recherchenbericht gilt

Nummer der Anmeldung: EP 97 11 0533

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int.Cl.6) |
|---|---|---|---|
| D,A | E. V. DEHMLOW ET AL: "Azuleno(6,5-d)isoxazole: Derivate eines neuartigen Ringsystems" CHEMISCHE BERICHTE, Bd. 119, Nr. 10, 1986, Seiten 2956-2962, XP002046469 * Seite 2956; Beispiel 2 * --- | 1,2 | C07C229/50 C07C233/52 C07C233/91 C07C237/18 |
| A | CHEMICAL ABSTRACTS, vol. 116, no. 12, 30.März 1992 Columbus, Ohio, US; abstract no. 128342u, YUN SHAN LIN ET AL: "Acetylation of 2-amino-1,3-disubstituted azulenes" Seite 830; XP002046470 * Zusammenfassung * & HUAXUE, Bd. 48, Nr. 4, 1990, Seiten 303-308, --- -/-- | 1,2 | |

| RECHERCHIERTE SACHGEBIETE (Int.Cl.6) |
|---|
| C07C |

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche:

Unvollständig recherchierte Patentansprüche:

Nicht recherchierte Patentansprüche:

Grund für die Beschränkung der Recherche:

Siehe Ergänzungsblatt C

| Recherchenort | Abschlußdatum der Recherche | Prüfer |
|---|---|---|
| DEN HAAG | 11.November 1997 | Voyiazoglou, D |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C09)

Europäisches Patentamt

EUROPÄISCHER TEILRECHERCHENBERICHT

Nummer der Anmeldung
EP 97 11 0533

| EINSCHLÄGIGE DOKUMENTE | | | KLASSIFIKATION DER ANMELDUNG (Int.Cl.6) |
|---|---|---|---|
| Kategorie | Kennzeichnung des Dokuments mit Angabe, soweit erforderlich der maßgeblichen Teile | Betrifft Anspruch | |
| A | CHEMICAL ABSTRACTS, vol. 69, no. 17, 21.Oktober 1968<br>Columbus, Ohio, US;<br>abstract no. 67389b,<br>TETSUO NOZOE ET AL: "Triazolylazulene derivatives"<br>Seite 6297;<br>XP002046471<br>* Zusammenfassung *<br>& JP 46 000 000 A (SANKYO) 11.Januar 1968<br>--- | 1-3 | |
| A | DE 40 21 437 A (KOTOBUKI) 17.Januar 1991<br>* Seite 17-7; Ansprüche 1,12 *<br>----- | 1,3,4 | |
| | | | RECHERCHIERTE SACHGEBIETE (Int.Cl.6) |

EPO FORM 1503 03.82 (P04C12)

Europäisches
Patentamt

**UNVOLLSTÄNDIGE RECHERCHE**
**ERGÄNZUNGSBLATT C**

Nummer der Anmeldung
EP 97 11 0533

Vollständig recherchierte Ansprüche:
2

Unvollständig recherchierte Ansprüche:
1,3-5

Grund für die Beschränkung der Recherche:

Die Ansprüche sind so breit gefasst dass die internationale Suche im Sinne von Artikeln 83 & 84 eingeschränkt werden musste. Während der internationalen Suche wurde besonderes Gewicht auf die folgenden Anmeldung gelegt: Seiten 24-28 und Ansprüch 2.